# FASCICULE DE BREVET EUROPEEN

(11) **EP 2 661 422 B1**
(45) Date de publication et mention de la délivrance du brevet: **20.04.2016**
(21) Numéro de dépôt: 12703858.6
(22) Date de dépôt: 04.01.2012
(51) Int. Cl.: C07C 231/02, C07C 233/18, C07C 253/30, C07C 255/37, C07C 255/40

(54) **PROCÉDÉ ET INTERMÉDIAIRES DE SYNTHÈSE DE L'AGOMÉLATINE**
VERFAHREN UND ZWISCHENPRODUKTE ZUR HERSTELLUNG VON AGOMELATINE
METHOD AND INTERMEDIATE PRODUCTS FOR THE PREPARATION OF AGOMELATINE

(30) Priorité: 05.01.2011 FR 1100024
(43) Date de publication de la demande: 13.11.2013
(73) Titulaire: Les Laboratoires Servier, 92284 Suresnes Cedex (FR)
(72) Inventeur: ZARD, Samir, F-91190 Gif-Sur-Yvette (FR); SIRE, Béatrice, F-91120 Palaiseau (FR); BOUMEDIENE, Mehdi, F-94600 Choisy Le Roi (FR)
(86) Numéro de dépôt international: PCT/FR2012/000005
(87) Numéro de publication internationale: WO 2012/113999

(56) Documents cités:
- EP-A1- 0 447 285
- EP-A1- 1 564 202

## Description

La présente invention concerne un nouveau procédé de synthèse industriel de l'agomélatine ou *N*-[2-(7-méthoxy-1-naphtyl)éthyl]acétamide de formule (I) :

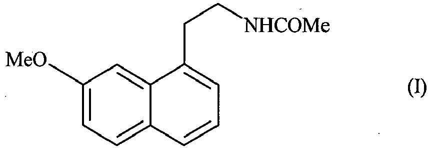

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone.
Dans le brevet EP 1 564 202, la demanderesse a mis au point une nouvelle voie de synthèse beaucoup plus performante et industrialisable, en seulement quatre étapes à partir de la 7-méthoxy-1-tétralone, et permettant d'obtenir l'agomélatine de façon très reproductible sous une forme cristalline bien définie.
Toutefois, la recherche de nouvelles voies de synthèse, en particulier à partir de matières premières moins onéreuses que la 7-méthoxy-1-tétralone, est toujours d'actualité.

La demanderesse a poursuivi ses investigations et mis au point un nouveau procédé de synthèse de l'agomélatine à partir de cyanure d'allyle et d'un dérivé xanthate: ces nouvelles matières premières présentent l'avantage d'être simples, aisément accessibles en grandes quantités avec des coûts moindres.
Cette voie de synthèse repose sur la mise en oeuvre de réactions radicalaires peu répandues et pourtant très efficaces. La transposition de ces réactions à l'échelle industrielle sur des réacteurs en flux continu est prometteuse dans la mesure où il devient plus facile de contrôler la propagation de la réaction en chaîne.
Ce nouveau procédé permet par ailleurs d'obtenir l'agomélatine de façon reproductible et sans nécessiter de purification laborieuse, avec une pureté qui est compatible avec son utilisation comme principe actif pharmaceutique. En effet, l'agomélatine peut ainsi être synthétisé en 6 étapes au cours desquelles seuls deux des intermédiaires sont isolés.

Plus spécifiquement, la présente invention concerne un procédé de synthèse industriel du composé de formule (I) : 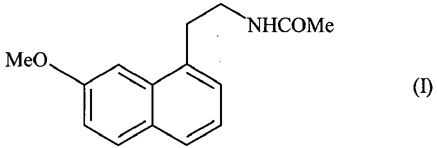 caractérisé en ce que l'on met en réaction le cyanure d'allyle de formule (II) : 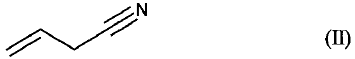 avec un composé de formule (III) en présence d'un amorceur radicalaire : 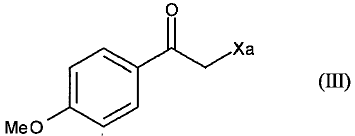 où Xa représente un groupement -S-C(S)-OR dans lequel R représente un groupement alkyle (C₁-C₆) linéaire ou ramifié,
pour conduire au composé de formule (IV) : 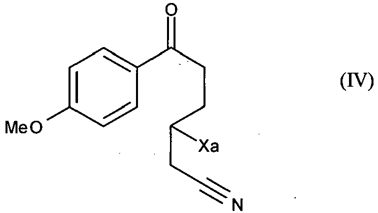 dans laquelle Xa est tel que défini précédemment,
ce dernier composé pouvant être optionnellement isolé avant d'être soumis à une réaction de cyclisation en présence d'un amorceur radicalaire pour former le composé de formule (V): 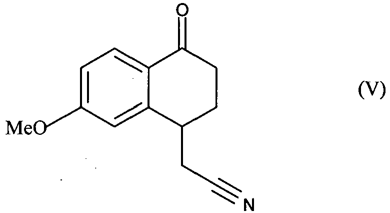 composé de formule (V) pouvant aussi être optionnellement isolé,
que l'on soumet à une réaction de réduction/déshydratation pour conduire au composé de formule (VI) : 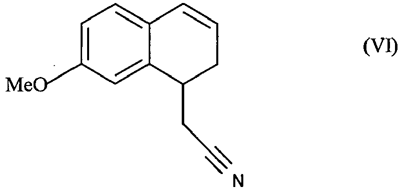 qui est ensuite soumis à une réaction d'aromatisation, pour conduire au composé de formule (VII) : 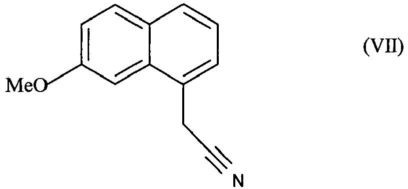 que l'on soumet à une réduction par l'hydrogène en présence de Nickel de Raney dans un milieu protique polaire et à une réaction avec l'anhydride acétique pour conduire au composé de formule (I) que l'on isole sous la forme d'un solide.

Dans un mode de réalisation préférée de l'invention, le composé de formule (VII) est ensuite soumis à une réduction par l'hydrogène en présence de Nickel de Raney dans un milieu éthanol ammoniacal, puis salifié avec de l'acide chlorhydrique pour conduire au composé de formule (VIII) : 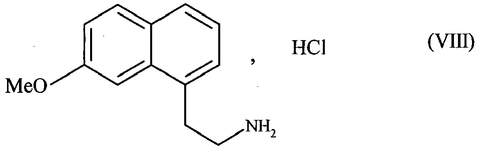 qui est successivement soumis à l'action d'acétate de sodium puis d'anhydride acétique pour conduire au composé de formule (I) que l'on isole sous la forme d'un solide.

Alternativement, le composé de formule (VII) peut être soumis à une réduction par l'hydrogène en présence de Nickel de Raney dans un milieu contenant de l'anhydride acétique dans un milieu protique polaire, pour conduire au composé de formule (I) que l'on isole sous la forme d'un solide.
Dans le composé de formule (III) préféré, Xa représente un groupe -S-C(S)-OC₂H₅.
Dans les procédés selon l'invention, l'initiation des réactions radicalaires est réalisée par voie thermique. De manière préférentielle, le milieu réactionnel est porté à une température comprise entre 50°C et 140°C. Plus préférentiellement encore, la cyclisation est réalisée à une température comprise entre 130 et 135°C.
Les peroxydes sont des amorceurs radicalaires particulièrement adaptés pour effectuer l'étape d'addition du composé de formule (II) sur le composé de formule (III), ou bien pour réaliser la cyclisation du composé de formule (IV) en composé de formule (V). A titre d'exemples, on peut notamment citer le peroxyde de diisobutyryle, le peroxynéodécanoate de cumyle, le peroxynéodécanoate de *tert*-amyle, le peroxydicarbonate de di(2-éthylhexyle), le peroxynéodécanoate de *tert*-butyle, le peroxydicarbonate de dibutyle, le peroxydicarbonate de dicétyle, le peroxydicarbonate de dimyristyle, le peroxynéoheptanoate de *tert*-butyle, le peroxypivalate de *tert*-amyle, le peroxyde de didécanoyle, le peroxy-2-éthylhexanoate de *tert*-amyle, le peroxyisobutyrate de *tert*-butyle, le 1,4-di(*tert*-butylperoxycarbo)cyclohexane, le peroxyacétate de *tert*-butyle, le peroxybenzoate de *tert*-butyle, le peroxyde de di-*tert*-amyle, le peroxyde de *tert*-butyle cumyle, le peroxyde de bis-tertiobutyle, le peroxyde de dicumyle, le peroxyde de dilauroyle (DLP) ou le peroxydicarbonate de di(4-*tert*-butylcyclohexyle).
De manière préférentielle, la réaction est amorcée en présence de peroxyde de dilauroyle.

La quantité de peroxyde de dilauroyle utilisée lors de la cyclisation est préférentiellement comprise entre 1 et 2,5 équivalents.
Dans un mode de réalisation préférée de l'invention, le peroxyde de dilauroyle est additionné de manière fractionnée dans le milieu.

Les réactions d'addition et/ou de cyclisation ont lieu dans un solvant utilisé classiquement en chimie radicalaire comme comme le 1,2-dichloroéthane, le dichlorométhane, le benzène, le toluène, le trifluorométhylbenzène, le chlorobenzène, l'hexane, le cyclohexane, l'heptane, l'octane, l'acétate d'éthyle, l'alcool tertiobutylique, et leurs mélanges.
On utilise préférentiellement l'acétate d'éthyle dans l'étape d'addition du composé de formule (II) sur le composé de formule (III), tandis que la cyclisation du composé de formule (IV) en composé de formule (V) est avantageusement réalisée dans le chlorobenzène, l'acétate d'éthyle, ou le butyrate d'éthyle. Dans ce dernier cas, le chlorobenzène est plus particulièrement préféré.

La transformation du composé de formule (V) en composé de formule (VI) est avantageusement réalisée en présence d'un acide de Lewis tel que l'isopropoxyde d'aluminium ou l'isopropoxyde de samarium. De plus, cette transformation a lieu préférentiellement dans un alcool (primaire ou secondaire), et plus préférentiellement encore dans l'isopropanol.

De manière préférentielle, une quantité catalytique d'acide *p*-toluènesulfonique est ajoutée au mélange une fois toute la tétralone (V) consommée à l'issue de la transformation du composé de formule (V) en composé de formule (VI).

L'aromatisation du composé (VI) est réalisée en présence d'une quinone, et de manière préférentielle en présence de 2,3-dichloro-5,6-dicyano-1,4-benzoquinone (DDQ) ou de tétrachlorobenzoquinone (TCQ). De manière plus préférentielle encore, l'aromatisation est effectuée en présence de TCQ au reflux du toluène.

Le composé de formule (II) est accessible à l'homme du métier par des réactions chimiques classiques et/ou décrites dans la littérature.

Ce procédé est particulièrement intéressant pour les raisons suivantes :
- il permet d'obtenir à l'échelle industrielle le composé de formule (I) avec de bons rendements à partir d'une matière première simple et peu onéreuse ;
- seuls les intermédiaires de formule (VI) et (VII) nécessitent une étape de purification et d'isolement.
Les composés de formule (V) et (VI) obtenus selon le procédé de l'invention sont nouveaux et utiles en tant qu'intermédiaires de synthèse de l'agomélatine.
Les exemples ci-dessous illustrent l'invention, mais ne la limitent en aucune façon.
Afin de bien valider le chemin réactionnel, les intermédiaires de synthèse ont été systématiquement isolés et caractérisés. Toutefois, il est possible d'optimiser considérablement les procédés en limitant le nombre d'intermédiaires isolés. Ainsi, l'exemple 2 détaillé ci-dessous correspond au même chemin réactionnel que celui emprunté à l'exemple 1, à la différence près que seuls le (7-méthoxy-1,2-dihydro-1-naphtalényl)acétonitrile et le (7-méthoxy-1 -naphtyl)acétonitrile ont été isolés.

### Exemple 1 : N-[2-(7-Méthoxy-1-naphtyl)éthyl]acétamide

### Stade A : Dithiocarbonate de S-[1-(cyanométhyl)-4-(4-méthoxyphényl)-4-oxobutyll-O-éthyle

Une solution de cyanure d'allyle (4,8 mL, 60,0 mmol) et de dithiocarbonate *S*-[2-(4-méthoxyphényl)-2-oxoéthyl]-*O*-éthyle¹ (8,1 g, 30,0 mmol) dans l'acétate d'éthyle (30 mL) est portée au reflux pendant 15 minute sous atmosphère d'azote. On ajoute dans un premier temps une quantité de peroxyde de dilauroyle (10 mol%) dans la solution au reflux. Au bout d'1h30, une autre quantité de peroxyde de dilauroyle (5 mol%) est également introduite. Lorsque les réactifs ont été complètement consommés, le mélange est refroidi à température ambiante et concentré sous pression réduite. Le mélange brut est ensuite purifié par chromatographie sur colonne flash (éther de pétrole- acétate d'éthyle : 95-5 à 80-20) pour conduire au composé du titre sous la forme d'une huile avec un rendement de 98%.
¹Le dithiocarbonate *S*-[2-(4-méthoxyphényl)-2-oxoéthyl]-O-éthyle est obtenu selon le protocole décrit dans Batanero, B et al, J. Org. Chem. 2001, 66, 320.
¹H NMR (δ, ppm) 7.93 (m, 2H, CH-*4*), 6.93 (m, 2H, CH-*3*), 4.67-4.57 (m, 2H, CH₂-*13*), (CDCl₃, 400 MHz) 3.99 (m, 1H, CH-*9*), 3.87 (s, 3H, CH₃-1), 3.15 (t, 2H, J= 7.3 Hz, CH₂-7), 2.95 (dd, 2H, J = 17.0, 6.0 Hz, CH₂-1*0*), 2.41-2.31 (m, 1H, CH₂-*8*), 2.19-2.08 (m, 1H, CH₂-*8*), 1.41 (t, 3H, J= 7.1 Hz, CH₃-*14*).

### Stade B : (7-Méthoxy-4-oxo-1,2,3,4-tétrahydro-1-naphtalényl)acétonitrile

Le composé du stade A, engagé pour la suite sans avoir été purifié, est redissout dans le chlorobenzène (900 mL) et la solution est portée au reflux pendant 15 minute sous atmosphère d'azote. Puis, du peroxyde de dilauroyle est progressivement ajouté dans la solution au reflux (10 mol% toutes les 10 min). En fin de réaction, le mélange est refroidi à température ambiante et concentré sous pression réduite. De l'acétonitrile est alors introduit pour faire précipiter une grande partie des dérivés du peroxyde de dilauroyle. Le mélange est ensuite filtré, concentré sous pression réduite et purifié par chromatographie sur colonne flash (éther de pétrole - acétate d'éthyle : 60-40) pour conduire au composé du titre sous forme solide avec un rendement de 40%.
**HRMS** (EI, m/z) Calc.pour C₁₃H₁₃NO₂: 215.0946 ; Trouvé :215.0946.

### Stade C : (7-Méthoxy-1,2-dihydro-1-naphtalényl)acétonitrile

De l'isopropoxide d'aluminium (2,05 g, 10,0 mmol) est ajouté à une solution du composé obtenu au stade B (680 mg, 3,15 mmol) dans l'isopropanol (15 mL) à température ambiante. Le mélange réactionnel est porté au reflux. Lorsque les réactifs ont été complètement consommés, on y ajoute quelques cristaux d'acide *p*-toluènesulfonique monohydraté et un appareil de Dean-Stark est installé au sommet du ballon. Le mélange est de nouveau porté au reflux pendant 1 heure, durant laquelle l'isopropanol est progressivement remplacé par du toluène à l'aide du Dean-Stark. Une solution de HCl IN est ensuite ajoutée et les phases résultantes sont séparées. La phase aqueuse est extraite avec de l'acétate d'éthyle, tandis que les phases organiques sont lavées avec une solution saturée de NaHCO₃, avec une solution saturée en NaCl, puis séchées sur MgSO₄, filtrées et concentrées sous pression réduite. Le résidu est purifié par chromatographie sur colonne (éther de pétrole - acétate d'éthyle : 80-20) pour conduire au produit du titre sous forme d'une huile avec un rendement de 85%.

### Stade D : (7-Méthoxy-1-naphtyl)acétonitrile

### Méthode A :

A une solution du composé obtenu au stade C (1,0 g, 5,0 mmol) dans le dichlorométhane (50 mL) à température ambiante est ajouté du DDQ (1,4 g, 6,0 mmol). Le mélange réactionnel est agité pendant 2 jours, puis nettoyé à l'aide d'une solution saturée en CO₃. La phase aqueuse est extraite avec de l'acétate d'éthyle, tandis que la phase organique est nettoyée avec une solution saturée en NaCl, séchée sur MgSO₄, filtrée et concentrée sous pression réduite. Le résidu est purifié par chromatographie sur colonne (éther de pétrole - acétate d'éthyle : 80-20) pour conduire au produit du titre sous forme solide avec un rendement de 55%.

### Méthode B :

A une solution de TCQ (615 mg, 2,5 mmol) dans le toluène (1,5 mL) chauffée à 80°C est ajouté le composé obtenu au stade C (462 mg, 2,3 mmol) dissout dans le toluène (3,5 mL). Le mélange est ensuite porté au reflux pendant 2.5 h, puis dilué dans l'eau et extrait avec de l'éther de pétrole. La phase organique est nettoyée avec une solution de NaOH (30%wt) et avec de l'eau, puis séchée sur MgSO₄, filtrée et concentrée sous pression réduite. Le résidu est purifié par chromatographie sur colonne (éther de pétrole - acétate d'éthyle : 80-20) pour conduire au produit du titre sous forme solide avec un rendement de 61%.
**HRMS** (EI, m/z) Calc.pour C₁₃H₁₁NO : 197.0841 ; Trouvé : 197.0838.

### Stade E : N-[2-(7-Méthoxy-1-naphtyl)éthyl]acétamide

La réaction a été réalisée sur une plus grosse charge afin d'optimiser le rendement obtenu : Dans un réacteur de 8 L sont introduits 136 g de Nickel de Raney, 2,06 L d'éthanol et 0,23 L d'eau. Sous agitation à 70°C et sous 30 bars d'hydrogène, le composé obtenu au Stade D (0,8 kg) en solution dans l'anhydride acétique (2,4 L) est ajouté lentement. A la fin de l'addition, le milieu réactionnel est agité 1 heure sous hydrogène à 30 bars, puis le réacteur est décompressé, et les jus sont filtrés. Après concentration du milieu, on cristallise le résidu dans un mélange éthanol/eau 35/65 pour conduire au produit du titre avec un rendement de 89% et une pureté chimique supérieure à 99%.

### Point de fusion : 108°C

### Exemple 2 : N-[2-(7-Méthoxy-1-naphtyl)éthyl] acétamide

### Stade A : (7-Méthoxy-1,2-dihydro-1-naphtalényl acétonitrile

Une solution de cyanure d'allyle (6.75 mL, 84.0 mmol) et de dithiocarbonate *S*-[2-(4-méthoxyphényl)-2-oxoéthyl]-*O*-éthyle¹ (11.3 g, 42.0 mmol) dans l'acétate d'éthyle (45 mL) est portée au reflux pendant 15 minutes sous atmosphère d'azote. On ajoute dans un premier temps une quantité de peroxyde de dilauroyle (10 mol%) dans la solution au reflux. Au bout d'1h30, une autre quantité de peroxyde de dilauroyle (5 mol%) est également introduite. Lorsque les réactifs ont été complètement consommés, le mélange est refroidi à température ambiante et concentré sous pression réduite. Le mélange brut est redissout dans le chlorobenzène (640 mL) et la solution est portée au reflux pendant 15 minutes sous atmosphère d'azote. Puis, du peroxyde de dilauroyle est progressivement ajouté dans la solution au reflux (10 mol% toutes les 10 min). En fin de réaction, le mélange est refroidi à température ambiante et concentré sous pression réduite. De l'acétonitrile est alors introduit pour faire précipiter une grande partie des dérivés du peroxyde de dilauroyle. Le mélange est ensuite filtré, concentré sous pression réduite. La moitié de l'huile brute ainsi obtenue est redissoute dans l'isopropanol (100 mL) à température ambiante en présence d'isopropoxide d'aluminium (13.6 g, 66.6 mmol). Le mélange réactionnel est porté au reflux. Lorsque les réactifs ont été complètement consommés, on y ajoute quelques cristaux d'acide *p*-toluènesulfonique monohydraté et un appareil de Dean-Stark est installé au sommet du ballon. Le mélange est de nouveau porté au reflux pendant 2 heures, durant lesquelles l'isopropanol est progressivement remplacé par du toluène à l'aide du Dean-Stark. Une solution de HCl 1N est ensuite ajoutée et les phases résultantes sont séparées. La phase aqueuse est extraite avec de l'acétate d'éthyle, tandis que les phases organiques sont nettoyées avec une solution saturée de NaHCO₃, avec une solution saturée en NaCl, puis séchées sur MgSO₄, filtrées et concentrées sous pression réduite. Le résidu est purifié par chromatographie sur colonne (éther de pétrole - acétate d'éthyle : 80-20) pour conduire au produit du titre sous forme d'une huile avec un rendement de 24%.
**HRMS** (EI, m/z) Calc.pour C₁₃H₁₃NO: 199.0997 ; Trouvé : 199.1001.

### Stade B : (7-Méthoxy-1-naphtyl)acétonitrile

On procède de manière analogue au stade D de l'Exemple 1.

### Stade C : N-[2-(7-Méthoxy-1-naphtyl)éthyl]acétamide

On procède de manière analogue au stade E de l'Exemple 1.

## Revendications

1. Procédé de synthèse industriel du composé de formule (I) : 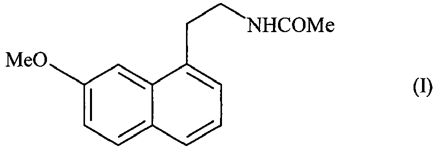 **caractérisé en ce que** l'on met en réaction le cyanure d'allyle de formule (II) : 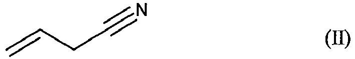 avec un composé de formule (III) en présence d'un amorceur radicalaire : 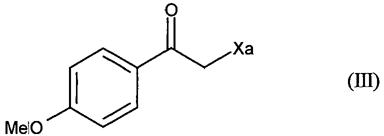 où Xa représente un groupement -S-C(S)-OR dans lequel R représente un groupement alkyle (C₁-C₆) linéaire ou ramifié,
pour conduire au composé de formule (IV) : 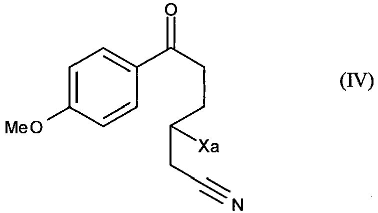 dans laquelle Xa est tel que défini précédemment,
ce dernier composé pouvant être optionnellement isolé avant d'être soumis à une réaction de cyclisation en présence d'un amorceur radicalaire pour former le composé de formule (V) : 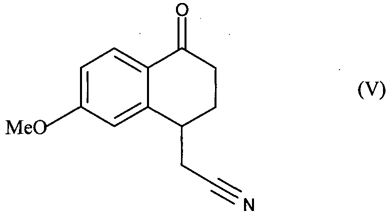 composé de formule (V) pouvant aussi être optionnellement isolé,
que l'on soumet à une réaction de réduction/déshydratation pour conduire au composé de formule (VI) : 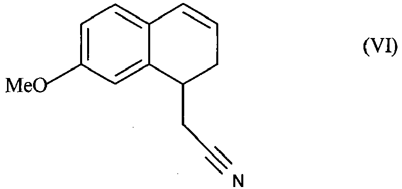 qui est ensuite soumis à une réaction d'aromatisation, pour conduire au composé de formule (VII) : 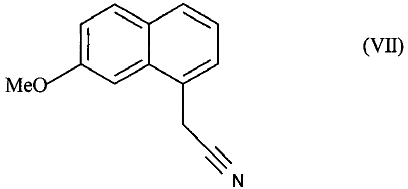 que l'on soumet à une réduction par l'hydrogène en présence de Nickel de Raney dans un milieu protique polaire et à une réaction avec l'anhydride acétique pour conduire au composé de formule (I) que l'on isole sous la forme d'un solide.

2. Procédé de synthèse du composé de formule (I) selon la revendication 1 **caractérisé en ce que** le composé de formule (VII) est ensuite soumis à une réduction par l'hydrogène en présence de Nickel de Raney dans un milieu éthanol ammoniacal, puis salifié avec de l'acide chlorhydrique pour conduire au composé de formule (VIII) : 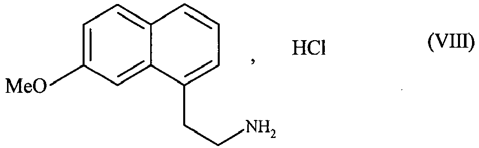 qui est successivement soumis à l'action d'acétate de sodium puis d'anhydride acétique pour conduire au composé de formule (I) que l'on isole sous la forme d'un solide.

3. Procédé de synthèse du composé de formule (I) selon la revendication 1 **caractérisé en ce que** le composé de formule (VII) est soumis à une réduction par l'hydrogène en présence de Nickel de Raney dans un milieu contenant de l'anhydride acétique dans un milieu protique polaire, pour conduire au composé de formule (I) que l'on isole sous la forme d'un solide.

4. Procédé de synthèse du composé de formule (I) selon la revendication 1, **caractérisé en ce que** le groupement Xa = -S-C(S)-OC₂H₅.

5. Procédé de synthèse du composé de formule (I) selon la revendication 1, **caractérisé en ce que** les réactions radicalaires sont initiées par voie thermique à une température comprise entre 50 et 140°C.

6. Procédé de synthèse du composé de formule (I) selon la revendication 1, **caractérisé en ce que** la cyclisation du composé de formule (IV) est réalisée à une température comprise entre 130 et 135°C.

7. Procédé de synthèse du composé de formule (I) selon la revendication 1, **caractérisé en ce que** l'étape d'addition du composé de formule (II) sur le composé de formule (III) et celle de cyclisation du composé de formule (IV) sont amorcées en présence de peroxyde de dilauroyle.

8. Procédé de synthèse du composé de formule (I) selon la revendication 1, **caractérisé en ce que** l'étape d'addition du composé de formule (II) sur le composé de formule (III) a lieu dans le chlorobenzène.

9. Procédé de synthèse du composé de formule (I) selon la revendication 1, **caractérisé en ce que** l'étape de cyclisation de l'adduit de formule (IV) en composé de formule (V) a lieu dans l'acétate d'éthyle.

10. Procédé de synthèse selon la revendication 1 **caractérisé en ce que** la transformation du composé de formule (V) en composé de formule (VI) est réalisée en présence d'isopropoxyde d'aluminium.

11. Procédé de synthèse selon la revendication 1 **caractérisé en ce que** la transformation du composé de formule (V) en composé de formule (VI) est réalisée dans l'isopropanol.

12. Procédé de synthèse selon la revendication 1 **caractérisé en ce qu'**une quantité catalytique d'acide *p*-toluènesulfonique est ajoutée au mélange à l'issue de la transformation du composé de formule (V) en composé de formule (VI).

13. Procédé de synthèse selon la revendication 1 **caractérisé en ce que** l'aromatisation du composé de formule (VI) est réalisée en présence d'une quinone.

14. Procédé de synthèse selon la revendication 1 **caractérisé en ce que** l'aromatisation du composé de formule (VI) est réalisée en présence de tétrachlorobenzoquinone TCQ au reflux du toluène.

15. Composé de formule (V) selon la revendication 1 utile en tant qu'intermédiaire de synthèse de l' agomélatine de formule (I) selon la revendication 1.

16. Utilisation du composé de formule (V) selon la revendication 15 dans la synthèse de l' agomélatine de formule (I) selon la revendication 1.

17. Composé de formule (VI) selon la revendication 1 utile en tant qu'intermédiaire de synthèse de l'agomélatine de formule (I) selon la revendication 1.

18. Utilisation du composé de formule (VI) selon la revendication 17 dans la synthèse de l'agomélatine de formule (I) selon la revendication 1.

## Patentansprüche

1. Verfahren zur industriellen Synthese der Verbindung der Formel (I): 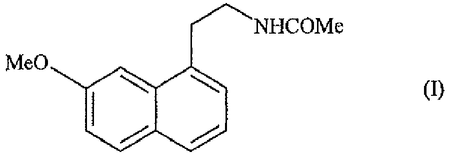 **dadurch gekennzeichnet, dass** man Allylcyanid der Formel (II): 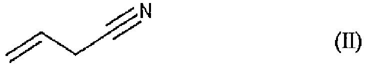 in Gegenwart eines Radikalinitiators mit einer Verbindung der Formel (III): 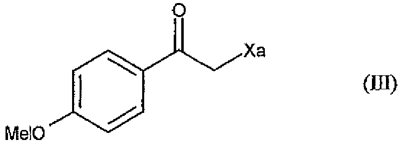 worin Xa eine Gruppe -S-C(S)-OR darstellt, worin R eine geradkettige oder verzweigte (C₁-C₆)-Alkylgruppe bedeutet,
umsetzt zur Bildung der Verbindung der Formel (IV): 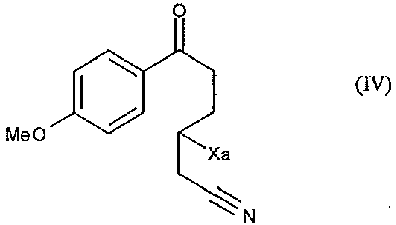 in der Xa die oben angegebene Bedeutung besitzt,
welche letztere Verbindung gegebenenfalls isoliert werden kann, bevor sie einer Cyclisierungsreaktion in Gegenwart eines Radikalinitiators unterworfen wird zur Bildung der Verbindung der Formel (V): 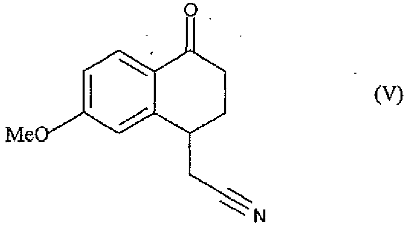 welche Verbindung der Formel (V) ebenfalls gegebenenfalls isoliert werden kann, welche man einer Reduktions-/Dehydratisierungsreaktion unterwirft zur Bildung der Verbindung der Formel (VI): 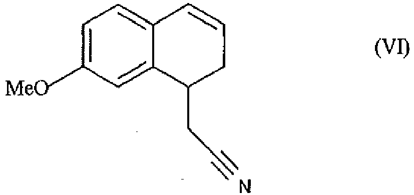 welche anschließend einer Aromatisierungsreaktion unterworfen wird zur Bildung der Verbindung der Formel (VII): 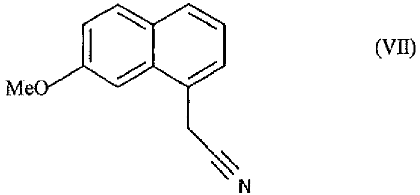 welche man in Gegenwart von Raney-Nickel in einem polaren protischen Medium einer Reduktion mit Wasserstoff und einer Reaktion mit Essigsäureanhydrid unterwirft zur Bildung der Verbindung der Formel (I), welche man in Form eines Feststoffs isoliert.

2. Verfahren zur Synthese der Verbindung der Formel (I) nach Anspruch 1, **dadurch gekennzeichnet, dass** die Verbindung der Formel (VII) anschließend in Gegenwart von Raney-Nickel in einem Ammoniak/Ethanol-Medium einer Reduktion mit Wasserstoff unterworfen wird und anschließend mit Chlorwasserstoffsäure einer Salzbildung unterzogen wird zur Bildung der Verbindung der Formel (VIII): 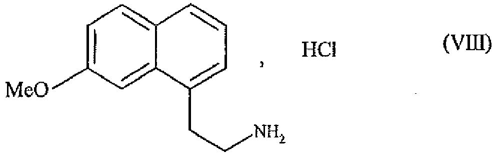 welche anschließend der Einwirkung von Natriumacetat und dann Essigsäureanhydrid unterzogen wird zur Bildung der Verbindung der Formel (I), welche man in Form eines Feststoffs isoliert.

3. Verfahren zur Synthese der Verbindung der Formel (I) nach Anspruch 1, **dadurch gekennzeichnet, dass** die Verbindung der Formel (VIII) in Gegenwart von Raney-Nickel in einem Medium, welches Essigsäureanhydrid in einem polaren protischen Medium enthält, einer Reduktion mit Wasserstoff unterzogen wird zur Bildung der Verbindung der Formel (I), welche man in Form eines Feststoffs isoliert.

4. Verfahren zur Synthese der Verbindung der Formel (I) nach Anspruch 1, **dadurch gekennzeichnet, dass** die Gruppe Xa = -S-C(S)-OC₂H₅ bedeutet.

5. Verfahren zur Synthese der Verbindung der Formel (I) nach Anspruch 1, **dadurch gekennzeichnet, dass** die radikalischen Reaktionen auf thermischem Wege bei einer Temperatur zwischen 50 und 140 °C initialisiert werden.

6. Verfahren zur Synthese der Verbindung der Formel (I) nach Anspruch 1, **dadurch gekennzeichnet, dass** die Cyclisierung der Verbindung der Formel (IV) bei einer Temperatur zwischen 130 und 135 °C durchgeführt wird.

7. Verfahren zur Synthese der Verbindung der Formel (I) nach Anspruch 1, **dadurch gekennzeichnet, dass** die Stufe der Addition der Verbindung der Formel (II) an die Verbindung der Formel (III) und die der Cyclisierung der Verbindung der Formel (IV) in Gegenwart von Dilauroylperoxid initialisiert werden.

8. Verfahren zur Synthese der Verbindung der Formel (I) nach Anspruch 1, **dadurch gekennzeichnet, dass** die Stufe der Addition der Verbindung der Formel (II) an die Verbindung der Formel (III) in Chlorbenzol durchgeführt wird.

9. Verfahren zur Synthese der Verbindung der Formel (I) nach Anspruch 1, **dadurch gekennzeichnet, dass** die Stufe der Cyclisierung des Addukts der Formel (IV) zu der Verbindung der Formel (V) in Ethylacetat durchgeführt wird.

10. Verfahren zur Synthese nach Anspruch 1, **dadurch gekennzeichnet, dass** die Umwandlung der Verbindung der Formel (V) in die Verbindung der Formel (VI) in Gegenwart von Aluminiumisopropylat durchgeführt wird.

11. Verfahren zur Synthese nach Anspruch 1, **dadurch gekennzeichnet, dass** die Umwandlung der Verbindung der Formel (V) in die Verbindung der Formel (VI) in Isopropanol durchgeführt wird.

12. Verfahren zur Synthese nach Anspruch 1, **dadurch gekennzeichnet, dass** eine katalytische Menge *p*-Toluolsulfonsäure am Ende der Umwandlung der Verbindung der Formel (V) in die Verbindung der Formel (VI) zu der Mischung zugegeben wird.

13. Verfahren zur Synthese nach Anspruch 1, **dadurch gekennzeichnet, dass** die Aromatisierung der Verbindung der Formel (VI) in Gegenwart eines Chinons durchgeführt wird.

14. Verfahren zur Synthese nach Anspruch 1, **dadurch gekennzeichnet, dass** die Aromatisierung der Verbindung der Formel (VI) in Gegenwart von Tetrachlorbenzochinon TCQ am Rückfluss in Toluol durchgeführt wird.

15. Verbindung der Formel (V) nach Anspruch 1, nützlich als Zwischenprodukt bei der Synthese von Agomelatin der Formel (I) nach Anspruch 1.

16. Verwendung der Verbindung der Formel (V) nach Anspruch 15 bei der Synthese von Agomelatin der Formel (I) nach Anspruch 1.

17. Verbindung der Formel (VI) nach Anspruch 1, nützlich als Zwischenprodukt bei der Synthese von Agomelatin der Formel (I) nach Anspruch 1.

18. Verwendung der Verbindung der Formel (VI) nach Anspruch 17 bei der Synthese von Agomelatin der Formel (I) nach Anspruch 1.

## Claims

1. Process for the industrial synthesis of the compound of formula (I): 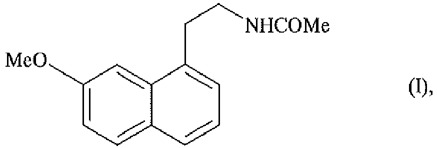 **characterised in that** allyl cyanide of formula (II): 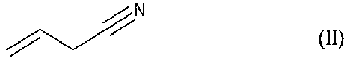 is reacted, in the presence of a free radical initiator, with a compound of formula (III): 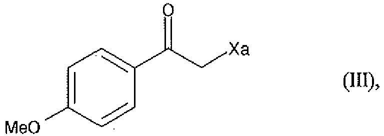 wherein Xa represents a group -S-C(S)-OR in which R represents a linear or branched (C₁-C₆)alkyl group,
to yield the compound of formula (IV): 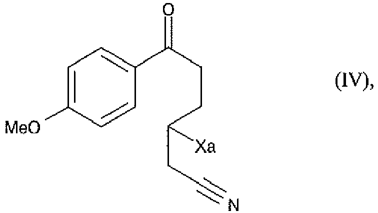 wherein Xa is as defined hereinbefore,
it being possible for this latter compound optionally to be isolated, before being subjected to a cyclisation reaction in the presence of a free radical initiator in order to form the compound of formula (V): 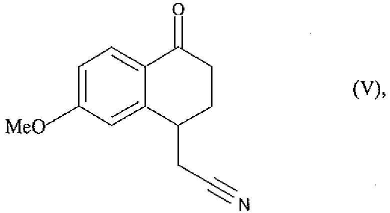 which compound of formula (V) also optionally may be isolated,
which is subjected to a reduction-dehydration reaction to yield the compound of formula (VI): 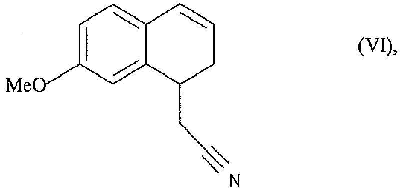 which is then subjected to an aromatisation reaction to yield the compound of formula (VII): 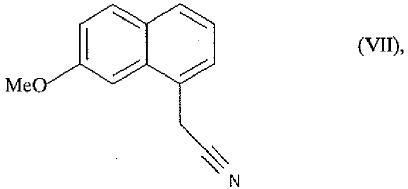 which is subjected to reduction using hydrogen in the presence of Raney nickel in a polar protic medium and to a reaction with acetic anhydride to yield the compound of formula (I), which is isolated in the form of a solid.

2. Process for the synthesis of the compound of formula (I) according to claim 1, **characterised in that** the compound of formula (VII) is then subjected to reduction using hydrogen in the presence of Raney nickel in an ammoniacal ethanol medium and then converted into a salt using hydrochloric acid to yield the compound of formula (VIII): 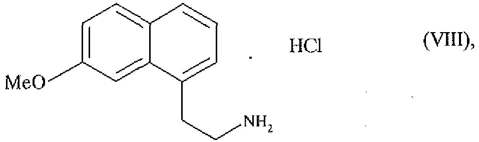 which is successively subjected to the action of sodium acetate and then acetic anhydride to yield the compound of formula (I), which is isolated in the form of a solid.

3. Process for the synthesis of the compound of formula (I) according to claim 1, **characterised in that** the compound of formula (VII) is subjected to reduction using hydrogen in the presence of Raney nickel in a medium comprising acetic anydride in a polar protic medium to yield the compound of formula (I), which is isolated in the form of a solid.

4. Process for the synthesis of the compound of formula (I) according to claim 1, **characterised in that** the group Xa = -S-C(S)-OC₂H₅.

5. Process for the synthesis of the compound of formula (I) according to claim 1, **characterised in that** the free radical reactions are initiated by thermal means at a temperature of from 50 to 140°C.

6. Process for the synthesis of the compound of formula (I) according to claim 1, **characterised in that** cyclisation of the compound of formula (IV) is carried out at a temperature of from 130 to 135°C.

7. Process for the synthesis of the compound of formula (I) according to claim 1, **characterised in that** the step of addition of the compound of formula (II) to the compound of formula (III) and that of cyclisation of the compound of formula (IV) are initiated in the presence of dilauroyl peroxide.

8. Process for the synthesis of the compound of formula (I) according to claim 1, **characterised in that** the step of addition of the compound of formula (II) to the compound of formula (III) is carried out in chlorobenzene.

9. Process for the synthesis of the compound of formula (I) according to claim 1, **characterised in that** the step of cyclisation of the adduct of formula (IV) to form the compound of formula (V) is carried out in ethyl acetate.

10. Synthesis process according to claim 1, **characterised in that** conversion of the compound of formula (V) into the compound of formula (VI) is carried out in the presence of aluminium isopropoxide.

11. Synthesis process according to claim 1, **characterised in that** conversion of the compound of formula (V) into the compound of formula (VI) is carried out in isopropanol.

12. Synthesis process according to claim 1, **characterised in that** a catalytic amount of *p*-toluenesulphonic acid is added to the mixture at the end of conversion of the compound of formula (V) into the compound of formula (VI).

13. Synthesis process according to claim 1, **characterised in that** aromatisation of the compound of formula (VI) is carried out in the presence of a quinone.

14. Synthesis process according to claim 1, **characterised in that** aromatisation of the compound of formula (VI) is carried out in the presence of tetrachlorobenzoquinone TCQ at the reflux of toluene.

15. Compound of formula (V) according to claim 1 for use as an intermediate in the synthesis of agomelatine of formula (I) according to claim 1.

16. Use of the compound of formula (V) according to claim 15 in the synthesis of agomelatine of formula (I) according to claim 1.

17. Compound of formula (VI) according to claim 1 for use as an intermediate in the synthesis of agomelatine of formula (I) according to claim 1.

18. Use of the compound of formula (VI) according to claim 17 in the synthesis of agomelatine of formula (I) according to claim 1.
